## Europäisches Patentamt

## European Patent Office

## Office européen des brevets

(19)

(11) Numéro de publication : **0 041 590**
**B1**

# FASCICULE DE BREVET EUROPÉEN

(12)

(45) Date de publication du fascicule du brevet :
**20.02.85**

(21) Numéro de dépôt : **80401012.2**

(22) Date de dépôt : **03.07.80**

(51) Int. Cl.⁴ : **G 01 N 33/76**, G 01 N 33/52

(54) **Réactif chimique indicateur pour le diagnostic "in vitro" de la grossesse.**

(30) Priorité : **06.06.80 FR 8012638**

(43) Date de publication de la demande :
**16.12.81 Bulletin 81/50**

(45) Mention de la délivrance du brevet :
**20.02.85 Bulletin 85/08**

(84) Etats contractants désignés :
**AT BE CH DE FR GB IT LI LU NL SE**

(56) Documents cités :
**GB-A- 2 018 989**
**US-A- 3 226 196**
**US-A- 3 298 787**
**US-A- 3 595 620**
**CHEMICAL ABSTRACTS, vol. 92, no. 5, 4 février 1980,**
**page 386, abrégé 37296b COLUMBUS, OHIO (US)**
**CHEMICAL ABSTRACTS, vol. 75, no. 2, 2 juillet 1971,**
**page 625, réf. 14558y COLUMBUS, OHIO (US)**

(73) Titulaire : **La Société Anstalt, dite FARMATEST**
**Vaduz (LI)**

(72) Inventeur : **de Gracia, Maria Reyes, née Gonzales**
**Res-Taval Apdo 6.D.Ramal 5 Collina Bello Monte**
**Caurimare Caracas (VE)**

(74) Mandataire : **Dupuy, René Gaston**
**Cabinet DUPUY & LOYER 14 rue La Fayette**
**F-75009 Paris (FR)**

## Description

### Domaine technique

L'invention a pour objet un réactif chimique indicateur pour le diagnostic « in vitro » de la grossesse d'un mammifère féminin, son procédé de fabrication et sa méthode d'utilisation.

### Technique antérieure

Il est bien connu d'utiliser la présence de la gonadotropine chorionique humaine (G. C. H.) dans l'urine ou le sérum des femmes enceintes à partir du 24ème jour de la grossesse avec une concentration maximale entre le 40ème et le 60ème jour, pour diagnostiquer l'état de grossesse.

On a tout d'abord effectué des tests biologiques en laboratoire en utilisant des animaux comme des rats et des lapins. Si l'exactitude de ces tests est satisfaisante, le temps et le matériel nécessaires pour leur mise en œuvre, et par conséquent leur coût, les rend impropres à une généralisation.

On a aussi proposé des tests immunologiques en provoquant une réaction entre la gonadotropine et ses anticorps. Ces tests ont aussi une précision satisfaisante, mais ils présentent les mêmes inconvénients que les tests biologiques : temps, difficulté de mise en œuvre, coût. L'emploi de tests chimiques directs sur les urines, à la fois rapide, plus précis et peu cher fut un progrès.

Le brevet américain La Vietes US-A-3 226 196 préconise l'emploi de deux réactifs A et B, à mélanger au moment du test. Celui-ci ne peut être effectué qu'en laboratoire, ne serait-ce que parce que le réactif A qui contient l'indicateur colorimétrique en l'occurrence un méthyle rouge, orange ou pourpre est constitué par de l'acide chlorhydrique concentré, le réactif B étant fortement alcalin (par exemple, hydroxyde de sodium ou potassium).

Le brevet américain US-A-3 595 620 déposé par Gordon le 28 octobre 1968 décrit et revendique un réactif chimique servant d'indicateur pour le diagnostic « in vitro » de la grossesse, en donnant une certaine coloration particulière aux urines auxquelles il est additionné. Ce réactif est constitué dans une solution tampon ayant un pH de 6 comportant du phosphate monobasique de potassium et d'hydroxyde de sodium, soit par du pourpre de Bromocresol soit du rouge de chlorophénol.

A ce réactif, on reproche le manque de fiabilité des résultats influencés par des phénomènes étrangers et la possibilité de fausse interprétation de ceux-ci, du fait du faible écart de coloration existant souvent entre le résultat positif (pourpre), et le résultat négatif qui varie selon les urines (comme il sera ultérieurement expliqué), de la couleur naturelle jaune au brun-rouge, écart qui peut descendre à une centaine d'Angströms. On sait aussi (Chemicals Abstracts Vol. 92-1980 N° 92 : 378966) déterminer le pH d'un liquide physiologique au moyen de papiers tests imprégnés de rouge de méthyle ou de bromothymol bleu.

### Exposé de l'invention

L'invention se propose d'éliminer les aléas et d'améliorer l'appréciation des résultats obtenus avec un réactif du type Gordon en accroissant l'écart entre la coloration due à une réaction négative et la coloration afférente à une réaction positive.

La Demanderesse fit exécuter un grand nombre d'essais cliniques sur des urines provenant de femmes non enceintes. Ces essais révélèrent un nombre important de cas faux et douteux où il aurait été impossible d'être affirmatif dans un sens ou dans l'autre, si l'on n'avait connu préalablement la bonne réponse. Les contrôles effectués indiquèrent que dans tous les cas, les urines avaient un pH anormalement bas ou anormalement haut avec, pour conséquence, que les urines d'une femme non enceinte (test dont le résultat aurait dû être nettement négatif) viraient dans la gamme de tons rouge pourpre, voisine de celle de tons qui auraient dû correspondre au test positif. On sait qu'en général le pH des urines normales se situe entre 5,5 et 6,5. Or, il existe au moins un cas où ce facteur d'acido-alcalimétrie accuse une tendance fortement basique, c'est celui où la femme a intégré antérieurement un médicament à base de stéroïdes (contraceptif par exemple).

Gordon, dans son brevet US-A-3 595 620 loin de penser que la présence de ce type d'hormone faussait le résultat du test, avait imaginé se servir au contraire de cette propriété pour déceler l'omission de prise d'une pilule contraceptive.

En pratiquant ces tests sur des urines de femmes non enceintes, la Demanderesse a observé que l'ingestion de composés stéroïdiques n'était pas la seule à fausser le résultat du test, mais que des résultats positifs ou douteux étaient indûment obtenus lorsque la femme en cause avait intégré des aliments à haute teneur en acides (acide citrique ou ascorbique) ou à haute teneur en protéines ou alcools.

Il apparut donc à la Demanderesse que pour obtenir des résultats fiables, il était nécessaire que le réactif n'agisse sur la G. C. H. que dans des conditions déterminées et de caractéristiques constantes.

Il fut observé qu'une condition indispensable pour obtenir des résultats sincères était que le pH des urines soit modifié pour être compris entre environ 4 et 5.

La Demanderesse, constatant que les stéroïdes, l'acide citrique ou ascorbique, l'alcool et les protéines ont une action sur le réactif analogue à celle de la G. C. H. en déduisit que celle-ci a un facteur commun avec ces produits et comme le facteur commun entre ces produits est la présence dans leur composition de groupes carbonyles, il est évident que les urines d'une femme enceinte contiennent aussi ces groupes provenant d'une réaction « in vivo » de la G. C. H. qui libère ces groupes spécifiques par l'intermédiaire du métabolisme humain.

Par ailleurs, des études « in vitro » mettant en présence de la G. C. H. synthétique et le réactif objet de la présente invention ayant donné des résultats négatifs montrent à l'évidence que si la G. C. H. synthétique est un mélange d'isomères, la G. C. H. naturelle est elle, un seul isomère donnant des réactions sélectives. La Demanderesse a pensé reproduire « in vitro » le processus « in vivo » en ajoutant à une urine de femme non enceinte les composés suivants : stéroïdes, acide citrique ou acide ascorbique, ainsi que l'alcool et les protéines. Ces expériences ont donné un résultat altéré, c'est-à-dire, ni positif, ni négatif dans le test de grossesse.

Les conclusions de ces expériences indiquent que les groupes carbonyles sont intervenus dans la réaction d'une certaine manière, donnant une altération absolue (ni négative, ni positive).

Les résultats « in vivo » ne sont pas identiques aux résultats obtenus « in vitro », du fait que dans les expériences « in vivo », les composés sont soumis au processus métabolique du corps humain entre le moment de l'ingestion et l'élimination des composés précités.

On considère donc que la réaction se fait entre un groupe carbonyle présent dans l'urine et le réactif objet de cette invention, ce groupe provenant d'une réaction « in vivo » de la G. C. H. qui libère des groupes carbonyles spécifiques dans le métabolisme des femmes enceintes et que ces groupes spécifiques s'éliminent par l'urine où ils sont détectés par ledit réactif.

La présente invention vise donc, pour contrebalancer la présence éventuelle d'un produit perturbateur dans les urines à tester, d'introduire dans la formule d'un réactif du type Gordon, c'est-à-dire comportant un crésol halogéné, ou un chlorophénol halogéné, un constituant compensateur susceptible de corriger les anomalies du pH et à les ramener et maintenir entre 4 et 5.

Par ailleurs, l'expérience a montré que la coloration du test positif était renforcée lorsque pour composant colorimétrique on utilisait un mélange de crésol halogéné (comme le pourpre de bromocrésol) et de chlorophénol halogéné (comme le rouge de chlorophénol) les deux composants ayant entre eux un effet synergique influant sur le degré de la coloration du test positif, le résultat optimal étant atteint quand le pourpre de bromocrésol et le rouge de chlorophénol étaient entre eux réciproquement dans un rapport pondéral d'environ 55 et 45 % et de préférence dans le rapport 53 et 47 %.

La Demanderesse a trouvé que parmi tous ces corps compatibles, la primauté devait être donnée au rouge de méthyle (2-diméthylamino-phényl-nitrobenzoïque) et au bleu de bromothymol (3-3' dibromothymol sulfonaphtalène) car ces phénols eux-mêmes interviennent dans la modification de la longueur d'onde de la coloration.

Le rapport pondéral entre le rouge de méthyle et le bleu de bromothymol est avantageusement, respectivement de 1/2.

Meilleure manière de réaliser le réactif

Le réactif doit être préparé dans des conditions telles qu'il ne puisse être perturbé par des agents extérieurs ;
— préparation dans un milieu isolé où aucune opération étrangère à ladite opération ne doit être effectuée,
— ambiance dénuée d'humidité et stable,
— air ambiant à température allant de 20 à 25 °C,
— stérilité de cet air, de tous les meubles et instruments y compris vêtements de l'opérateur qui doit porter un masque également stérile,
— le mélange des ingrédients doit être parfait de façon à ce qu'il n'existe pas en fin de préparation un résidu, ce qui modifierait les résultats du test,
— le réactif obtenu doit être conservé dans des bouteilles de verre et de couleur sombre, et ces bouteilles doivent être hermétiquement fermées.

On obtient le réactif en mélangeant trois solutions composées séparément :

On commence par préparer une solution tampon (solution A) en plaçant dans un ballon gradué de 1 litre, 250 millilitres d'une solution 0,2 molar de phosphate monobasique de sodium à laquelle on ajoute 28 millilitres d'une solution 0,2 molar d'hydroxyde de sodium, et en complétant le contenu à 1 litre avec de l'eau distillée.

On prépare ensuite une solution colorante (solution B) en mélangeant par agitation 0,20 g de pourpre de bromocrésol, 0,18 g de rouge de chlorophénol dans 18 millilitres de la solution tampon A puis on complète avec cette même solution A jusqu'à concurrence de 700 millilitres.

On prépare enfin une solution C en dissolvant dans 250 millilitres de solution tampon A : 0,10 g de rouge de méthyle et 0,20 g de bleu de bromothymol et 0,12 g de dichlorophénol indophénol.

Le réactif résulte du mélange des solutions B et C que l'on complète avec de l'eau distillée jusqu'à concurrence d'un litre.

Le mode d'emploi du réactif précédemment obtenu en vue d'un test déterminant la grossesse est le suivant :

a) on prend un échantillon d'urine du matin de la personne à jeun,

b) on place l'urine dans un récipient sec, propre et hygiénique durant 15 minutes jusqu'à ce que l'urine acquiert la température ambiante. (Lorsque l'échantillon d'urine a été conservé dans un réfrigérateur, on doit le ramener à la température ambiante avant d'effectuer l'expérience. Dans ce cas, le temps d'attente avant d'être utilisé ne doit pas dépasser la limite de huit heures. Les échantillons réfrigérés ne doivent pas être gardés plus de deux jours. Les échantillons congelés peuvent être conservés jusqu'à 25 jours).

c) dans un récipient cylindrique incolore de 2 centimètres de diamètre à fond plat, on met 5 centilitres d'urine auxquels on mélange 2 gouttes du réactif (de 0,132 cm³ chacune), et on agite,

d) si le mélange prend une couleur bleu pourpre, rose pâle ou rose pourpre, le résultat est positif ; si on ajoute plus de 4 gouttes du réactif, les couleurs sus-citées s'intensifieront tout en restant dans la même gamme,

e) si le mélange reste d'une couleur de la gamme des jaunes ou des bruns clairs, c'est-à-dire une couleur de la gamme de l'urine testée avant mélange, à peine plus foncée que la couleur naturelle de cette urine, le résultat est négatif ; si on ajoute plus de 4 gouttes du réactif la couleur précitée s'intensifiera tout en restant dans la même gamme,

f) le résultat est obtenu en vingt secondes maximum.

Entre les longueurs d'onde des couleurs du test positif et du test négatif, il y a donc un écart se situant aux environ de 10 μm.

Il y a néanmoins lieu d'observer les précautions ci-après :

— on doit arrêter la prise de médicaments anticonceptionnels 48 heures avant d'effectuer ce test,

— pendant la nuit qui précède ce test, il faut éviter les agents acidifiants et les antiacides tels que : oranges, citrons, viandes, bicarbonate, médicaments effervescents, vinaigre ainsi que d'autres produits similaires,

— si on a pris du citrate de clomifère ou un autre médicament fertilisant pendant une brève période ou plus de deux semaines avant le test, il ne devrait pas se présenter d'interférences,

— pour faciliter la lecture et éviter toute confusion entre les couleurs, on doit observer le résultat obtenu contre un fond blanc et à la lumière naturelle.

Mode opératoire

Le réactif objet de la présente invention est capable de déceler la présence de la G. C. H. présente dans les urines dès 4 à 6 jours après le premier arrêt des règles.

La première expérience devra avoir lieu entre le 4ème et le 6ème jour après la constatation de cet arrêt et peut être renouvelée pendant une période de trois mois à dater de celui-ci.

La meilleure période pour pratiquer le test se situe entre le 4ème et le 17ème jour ainsi calculés.

A partir du 90ème jour, le taux de l'hormone baisse sensiblement entraînant par corollaire, la baisse de l'efficacité du réactif.

Enfin, il faut remarquer que la présence du rouge de méthyle et du bleu de bromothymol dans le réactif, non seulement ont les effets compensateurs sus-indiqués, dans les cas où un produit ingéré risquerait de fausser le résultat, mais encore, donnent une stabilité presque infinie au réactif, alors qu'en leur absence, celui-ci ne serait stable que deux ou trois mois seulement.

## Revendications

1. Réactif chimique colorimétrique servant d'indicateur pour le diagnostic « in vitro » de la grossesse et destiné à être ajouté à une quantité d'urine émise par un mammifère féminin objet du test, ledit réactif comportant, dans une solution tampon dont le pH est compris entre 5,2 et 6,6, comme colorant sensible à la présence dans ces urines de la gonadotropine chorionique humaine (G. C. H.), un crésol halogéné ou un phénol halogéné, caractérisé en ce qu'il comprend en outre au moins un composant destiné à maintenir le pH des urines entre 4 et 5.

2. Réactif chimique selon la revendication 1, caractérisé en ce que le composant destiné à maintenir et à stabiliser le pH des urines testées entre 4 et 5 est un mélange de rouge de méthyle (2-diméthylaminophénylnitrobenzoïque) et le bleu de bromothymol (3-3' de bromothymolsulfonaphtalène).

3. Réactif chimique selon la revendication 2, caractérisé en ce que le rouge de méthyle et le bleu de bromothymol sont respectivement dans le rapport pondéral d'environ 1/2.

4. Réactif chimique selon la revendication 3, caractérisé en ce qu'il comprend une solution obtenue en faisant dissoudre 0,10 g de rouge de méthyle, 0,20 g de bleu de bromothymol, et 0,12 g de dichlorophénol indophénol dans 250 millilitres de ladite solution tampon.

5. Réactif selon la revendication 1, caractérisé en ce que ce colorant est un mélange de crésol halogéné et de phénol halogéné.

6. Réactif selon la revendication 5, dans lequel le crésol halogéné est le pourpre de bromocrésol (5'-5'' dibromo-o-crésolsulphonaphtalène) et le chlorophénol halogéné est le rouge de chlorophénol (3'-3'' dichlorophénolsulfonaphtalène), caractérisé en ce que le pourpre de bromocrésol et le rouge de dichlorophénol sont respectivement dans un rapport pondéral d'environ 55 et 45 % et de préférence dans le rapport 53-47 %.

7. Réactif chimique selon l'une des revendications précédentes, dans lequel la solution tampon est obtenue en mélangeant 250 millilitres de solution 0,2 molar de phosphate monobasique de sodium, de 25 à 30 millilitres (de préférence 28 millilitres) de solution 0,2 molar d'hydroxyde de sodium et en complétant pour obtenir un litre de solution tampon, avec de l'eau distillée, caractérisé en ce que l'on ajoute de 15 à 20 millilitres de cette solution tampon à 0,20 g de pourpre de bromocrésol, et 0,18 g de rouge de chlorophénol.

8. Réactif chimique selon l'une des revendications précédentes, caractérisé en ce que l'on ajoute le mélange obtenu selon la revendication 4 avec le mélange obtenu selon la revendication 7, et que l'on complète avec de l'eau distillée à concurrence d'un volume d'un litre.

9. Test pour diagnostic « in vitro » de la grossesse selon lequel on met en présence le réactif colorimétrique avec l'urine à tester, caractérisé en ce que l'on mélange 1 à 2 gouttes, d'environ un volume de 0,132 cm³ chacune, du réactif obtenu selon la revendication 8 avec environ 5 cl de cette urine.

## Claims

1. Chemical reagent for use as an indicator in the « in vitro » diagnosis of pregnancy, adapted to be added to an amount of urine collected from a female mammal under test, said reagent comprising, in a buffer solution having a pH value within the range of 5.2 to 6.6, a dye responsive to the presence of human chorionic gonadotropin (H. C. G.) in said urine, characterised in that further comprises at least one component adapted to maintain the urine pH between 4 and 5.

2. The chemical reagent of claim 1, characterised in that the component used for maintaining and stabilizing the pH of the tested urines between 4 and 5 is a mixture of (2 dimethylaminophenyl) methyl red and (3-3' dibromothymol sulphonaphtalene) bromothymol blue.

3. The reagent of claim 2, wherein said methyl red and bromothymol blue are in the ratio of about 1/2 by weight.

4. The reagent of claim 3, which comprises a solution obtained by dissolving 0.10 g of methyl red, 0.20 g of bromothymol blue and 0.12 g of dichlorophenol indophenol in 250 millilitres of said buffer solution.

5. The reagent of claim 1, wherein said colorant is a mixture of halogenated cresol and halogenated phenol.

6. Reagent according to claim 5, in which the halogenated cresol is bromocresol (5'-5" dibromo-o-cresolsulphonaphtalene) purple and the halogenated chlorophenol is chlorophenol (3'-3" dichlorosulphonaphtalene) red, characterised in that the ratio of said bromocresol purple to said dichlorophenol red is about 55 % and 45 % by weight and preferably 53 % and 47 % by weight, respectively.

7. The reagent of any of the preceding claims, wherein the buffer solution is obtained by mixing 250 millilitres of a 0.2 molar solution of sodium monobasic phosphate, 25 to 30 millilitres (preferably about 28 millilitres) of a 0.2 molar solution of sodium hydroxide with distilled water, 15 to 20 millilitres of said buffer solution being added to 0.20 g of bromocresol purple and 0.8 g of chlorophenol red.

8. The reagent of any of the preceding claims, characterised in that the mixture obtained according to claim 4 is added to the mixture obtained according to claim 7 and that the new mixture is completed with distilled water up to a volume of one litre.

9. A test for the « in vitro » diagnosis of pregnancy, in which the colorimetric reagent is brought together with the urine sample to be tested, characterised in that one or two drops (corresponding each to a volume of about 0.132 cm³) of the reagent of claim 8 are mixed with about 5 centilitres of the urine sample.

## Ansprüche

1. Kolorimetrisches Reagens als Indikator für den Schangerschaftsnachweis « in vitro », das einer von einem dem Test unterworfenen weiblichen Säugetier abgegebenen Harnmenge zugesetzt wird, wobei das genannte Reagens in einer Pufferlösung mit einem pH-Wert zwischen 5,2 und 6,6 als auf die Anwesenheit von Human-Choriongonadotropin (H. C. G.) in diesen Harnmengen ansprechenden Farbstoff ein halogenhaltiges Kresol oder Phenol enthält, dadurch gekennzeichnet, dass es ferner wenigstens eine Komponente umfasst, die dazu bestimmt ist, den pH-Wert der Harnproben zwischen 4 und 5 zu halten.

2. Kolorimetrisches Reagens nach Anspruch 1, dadurch gekennzeichnet, dass die Komponente zur Aufrechterhaltung und Stabilisierung des pH-Wertes der untersuchten Harnproben zwischen 4 und 5 ein Gemisch von Methylrot (2-Dimethylaminophenylbenzolkarbonsäure) und Bromthymolblau (3-3'-Bromthymolsulfonaphthalin) ist.

3. Kolorimetrisches Reagens nach Anspruch 2, dadurch gekennzeichnet, dass das Methylrot und das Bromthymolblau jeweils in einem gewichteten Verhältnis von etwa 1/2 zueinander stehen.

4. Kolorimetrisches Reagens nach Anspruch 3, dadurch gekennzeichnet, dass es eine Lösung enthält, die durch Auflösung von 0,10 g Methylrot, 0,20 g Bromthymolblau und 0,12 g Dichlorphenol-Indophenol in 250 Milliliter der genannten Pufferlösung erhalten wird.

5. Kolorimetrisches Reagens nach Anspruch 1, dadurch gekennzeichnet, dass dieser Farbstoff ein Gemisch aus halogenhaltigem Kresol und Phenol ist.

6. Kolorimetrisches Reagens nach Anspruch 5, in dem das halogenhaltige Kresol Bromkresolpurpur (5-5"-Dibrom-o-kresolsulfonaphthalin) und das halogenhaltige Chlorphenol Chlorphenolrot (3'-3"-Dichlorphenolsulfonaphthälin) ist, dadurch gekennzeichnet , dass der Bromkresolpurpur und das Dichlorphenolrot jeweils in einem gewichteten Verhältnis von etwa 55 und 45 % und vorzugsweise in einem Verhältnis von 53-47 % zueinander stehen.

7. Kolorimetrisches Reagens nach einem der vorstehenden Ansprüche 1 bis 6, in dem die Pufferlösung durch Vermischen von 250 Milliliter 0,2 molarer Mononatriumphosphatlösung, 25-30 (vorzugsweise 28 Milliliter) 0,2 molarer Natriumhydroxydlösung und durch Auffüllen mit destilliertem Wasser auf ein Liter Pufferlösung erhalten wird, dadurch gekennzeichnet, dass 15-20 Milliliter dieser Pufferlösung zu 0,20 g Bromkresolpurpur und 0,18 g Chlorphenolrot hinzugegeben werden.

8. Kolorimetrisches Reagens nach einem der vorstehenden Ansprüche 1 bis 7, dadurch gekennzeichnet, dass das nach Anspruch 4 erhaltene Gemisch mit dem nach Anspruch 7 erhaltenen Gemisch zugegeben und mit destilliertem Wasser auf ein Volumen von einem Liter aufgefüllt wird.

9. Test für Schwangerschaftsnachweis « in vitro », der darin besteht, dass das kolorimetrische Reagens mit dem zu untersuchenden Harn in Verbindung gebracht wird, dadurch gekennzeichnet, dass 1 bis 2 Tropfen des nach Anspruch 8 erhaltenen Reagenses mit einem Volumen von jeweils etwa 0,132 $cm^3$ mit etwa 5 cl dieser Harnprobe vermischt werden.